# EUROPEAN PATENT APPLICATION

(11) **EP 1 621 194 A1**
(43) Date of publication of application: **01.02.2006**
(21) Application number: 04808123.6
(22) Date of filing: 24.12.2004
(51) Int. Cl.: A61K 31/352, A61K 31/192, A61K 31/216, A61K 31/11, A61K 47/10, A61K 47/14, A61K 47/24, A61K 47/36, A61K 35/64, A61K 8/18, A61P 39/06, A23L 1/30

(54) **ACTIVE OXYGEN ELIMINATOR ORIGINATING IN NATURAL MATERIAL AND USE OF THE SAME**

(30) Priority: 16.01.2004 JP 2004008862
(71) Applicant: Nihon Propolis Co., Ltd., Nasushiobara-shi, Tochigi 325-0025 (JP)
(72) Inventor: MIDORIKAWA, Kiyoshi, c/o Nihon Propolis Co., Ltd, Nasushiobara-shi, Tochigi 325-0025 (JP); KADOTA, Shigetoshi, Toyama-shi, Toyama 930-0882 (JP); MATSUSHIGE, Katsumichi, Funabashi-shi, Chiba 273-0003 (JP)
(74) Representative: Albrecht, Thomas
(86) International application number: PCT/JP2004/019773
(87) International publication number: WO 2005/067918

(57) **Abstract**

An active oxygen eliminator derived from a natural substance, which comprises water, an organic solvent and an emulsifier and also comprises, as essential active components, p-coumarinic acid, 3,4-di-O-caffeoylquinic acid, 3,5-di-O-caffeoylquinic acid, 4-hydroxy-3-prenylcinnamic acid and 3,5,7-trihydroxy-4'-methoxyflavonol, a food comprising the active oxygen eliminator and a cosmetic comprising the active oxygen eliminator.

## Description

### Technical field

The present invention relates to an active oxygen eliminator derived from a natural substance and a use thereof. More specifically, the present invention relates to an active oxygen eliminator derived from a natural substance, which eliminator contains flavonoids or hydroxycinnamic acid derivatives as active components and is useful for foods and cosmetics, and a food and cosmetic containing the above active oxygen eliminator.

### Technical Background

Oxygen is a substance essential for supporting life of a living organism, such as metabolical energy production, and the like. However, due to various reactions inside and outside a living organism such as some immune reactions or irradiation with ultraviolet rays or radiation, oxygen produces highly reactive active oxygen species (super oxide anion: O₂⁻, peroxide: H₂O₂, hydroxyradical: OH⁻, singlet oxygen: ¹O₂, etc.) and free radicals. On one hand, these active oxygen species and free radicals work to deactivate foreign matters such as invasion bacteria, etc., for example, in an immune reaction, and on the other hand, it is known that they react with components of living organism to cause peroxidation of lipid and alteration of protein or nucleic acid, and cause various diseases or the promotion of aging. Particularly, a skin is the outermost layer of a body so that it is susceptible to active oxygen species or free radicals generated by external factors such as ultraviolet rays and radiation. It is known that when these are produced in the skin to excess, the formation of lipid peroxide by a radical reaction or the production of abnormal dyestuffs such as blotches, freckles, etc., in the skin are enhanced.

Super oxide anion O₂⁻ generated in a living organism by various causes is converted to H₂O₂ with an enzyme superoxide dismutase (to be abbreviated as "SOD" hereinafter), and the H₂O₂ is decomposed into water and oxygen by the activity of an enzyme catalase or glutathione peroxidase. In addition to these enzymes, anti-oxidative substances such as vitamin C, vitamin E, etc., derived from foods are present in a living organism, and these form a network for controlling radical reactions and oxidation reactions in the living organism and prevent various disorders caused by active oxygen and free radicals. As is known, however, when the balance of the above network is destroyed due to changes in surrounding and invivo environments, for example, infection with bacteria or virus, a change in the state of food ingestion or the state of nutrition, excess irradiation with ultraviolet ray, externally exerted various stresses, aging, senility, and the like, the balance of metabolism of active oxygen and free radicals produced in a living organism is destroyed. As a result, the amount of lipid peroxide increases, and there appear cosmetically impairing various symptoms such as dermatitis, blotches, wrinkles, eczema, freckles, and the like. Further, there are caused articular rheumatism, arteriosclerosis, diabetes, hepatitis, nephritis, cancer, and the like.

Further, with regard to foods and food additives to be ingested by people, it is also well known that lipid and other components contained therein undergo peroxidation or alteration due to oxidation caused by active oxygen or free radicals generated by the activity of ultraviolet ray or radiation as well as ordinary oxidation, and that it is undesirable for reasons of health to ingest such oxidized or altered products. Further, unsaturated-lipid-containing natural oils and fats, surfactants or the like are often incorporated into cosmetics or external preparations for skins, and it is known that some of them are susceptible to oxidation due to the activity of ultraviolet ray, or the like, and as a result, there is often caused an undesirable phenomenon such as discoloration, the occurrence of an offensive smell, or the like. It can be easily assumed that the amount of lipid peroxide generated in a skin by the above various causes increases due to the use of such a cosmetic base susceptible to oxidation and that the above cosmetically impairing various symptoms are hence amplified.

As a conventional antioxidant for cosmetics, foods, food additives and animal feeding stuffs, there are synthetic antioxidant such as dibutylhydroxytoluene (BHT), butylhydroxyanisol (BHA), ethoxyquin, and the like, and natural antioxidants such as ascorbic acid, vitamin E, and the like. While the above synthetic antioxidants are excellent in antioxidation effect, some of them have safety problems such as carcinogenicity, etc., and some of them are limited in use. Natural antioxidants are valuable with regard to safety, and yet they have a defect that their antioxidation effect is considerably poor as compared with any synthetic antioxidants.

In recent years, under the circumstances, searches have been actively made for substances or compositions having a free-radical-eliminating effect or an antioxidation effect from the viewpoint of perfume and cosmetic science, food science or pharmaceutical science, and as a result, a considerable number of active oxygen-free radical eliminators or antioxidants have come to be known. For example, there have been proposed an active oxygen eliminator containing baicalein that is a flavonoid component contained in scutellaria root (e.g., see JP-B-4-34969), an active oxygen eliminator or remover containing clove oil or dehydrodieugenol that is a component thereof (e.g., see JP-A-3-227938), and an active oxygen eliminator containing a walnut shell extract as an active component (e.g., see JP-A-7-69912).

Meanwhile, propolis has been conventionally known as a natural anti-bacterial substance or a health-building substance. The propolis is a dark-green-colored or brown-colored glutinous substance that honeybees make for the conservation of a honeycomb, and It is made of a mixture of an aggregate of gum matter, sap, a plant dyestuff-based substance, perfume oil, etc., which honeybees have collected from trees, with secretion, beeswax, etc., of the honeybees themselves. With regard to the above propolis, there have been made reports on various bioactivities such as antitumor activity, antioxidation activity, antiinflammation activity, antibacterial activity, and the like. Compounds having bioactivities include cinnamic acid derivatives, flavonoids, esters and other phenolic compounds, and with regard to a propolis originated from a subtropical region, there is a report on the presence of terpenes.

### Disclosure of the Invention

It is an object of the present invention to provide an active oxygen eliminator derived from a natural substance, which eliminator is highly capable of removing active oxygen or free radicals, is safe to a human body and is useful in various fields, and a use thereof. The present inventors have made diligent studies for achieving the above object, and have found that the above object can be achieved by a substance derived from a natural substance, which substance can be obtained, for example, by extracting powder of a raw propolis mass and contains a plurality of effective components such as flavonoids, a hydroxycinnamic acid derivative, other phenolic compounds, and the like. The present invention has been accordingly completed on the basis of the above finding.

That is, the present invention provides;
(1) an active oxygen eliminator derived from a natural substance, which comprises water, an organic solvent and an emulsifier and also comprises, as essential active components, p-coumarinic acid, 3,4-di-O-caffeoylquinic acid, 3,5-di-O-caffeoylquinic acid, 4-hydroxy-3-prenylsinnamic acid and 3,5,7-trihydroxy-4'-methoxyflavonol,
(2) an active oxygen eliminator derived from a natural substance as recited in the above (1), which further comprises, as active components, 3,5-diprenyl-4-hydroxycinnamic acid, kaempheride, beturetol, chlorogenic acid, ermanin, chrysin, caffeic acid, kaempherol and vanillin,
(3) an active oxygen eliminator derived from a natural substance as recited in the above (1) or (2), wherein the organic solvent is a polyhydric alcohol compound having 2 or more hydroxyl groups in a molecule,
(4) an active oxygen eliminator derived from a natural substance as recited in the above (1), (2) or (3), wherein the emulsifier is at least one member selected from a glycerin fatty acid ester, a polyglycerin fatty acid ester, a propylene glycol fatty acid ester, a sorbitan fatty acid ester, a sucrose fatty acid ester, lecithins and saponins,
(5) a food comprising the active oxygen eliminator derived from a natural substance recited in any one of the above (1) to (4), and
(6) a cosmetic comprising the active oxygen eliminator derived from a natural substance recited in any one of the above (1) to (4).

According to the present invention, there can be provided an active oxygen eliminator derived from a natural substance, which is highly capable of eliminating active oxygen and free radicals, which is safe to human bodies and which is useful in various fields, and there can be also provided foods and cosmetics comprising the above active oxygen eliminator each.

### Brief Description of Drawings

Fig. 1 shows a high-performance liquid chromatogram on one embodiment of an extract obtained from a raw propolis mass by using water as an extracting solvent.
Fig. 2 shows a high-performance liquid chromatogram on one embodiment of an extract obtained from a raw propolis mass by using glycerin as an extracting solvent.
Fig. 3 shows a high-performance liquid chromatogram on one embodiment of an extract obtained from a raw propolis mass by using a mixture of water with glycerin as an extracting solvent.
Fig. 4 shows a high-performance liquid chromatogram on one embodiment of an extract obtained from a raw propolis mass by using a mixture of water, glycerin and an emulsifier as an extracting solvent.

### Preferred Embodiments of the Invention

The active oxygen eliminator of the present invention is derived from a natural substance, and it comprises water, an organic solvent and an emulsifier and also comprises, as an active components, p-coumarinic acid (or p-coumarinic acid), 3,4-di-O-caffeoylquinic acid, 3,5-di-O-caffeoylquinic acid, 4-hydroxy-3-prenylcinnamic acid and 3,5,7-trihydroxy-4'-methoxyflavonol, which have the following chemical structures.

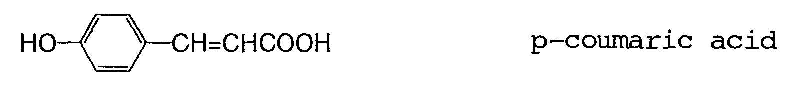

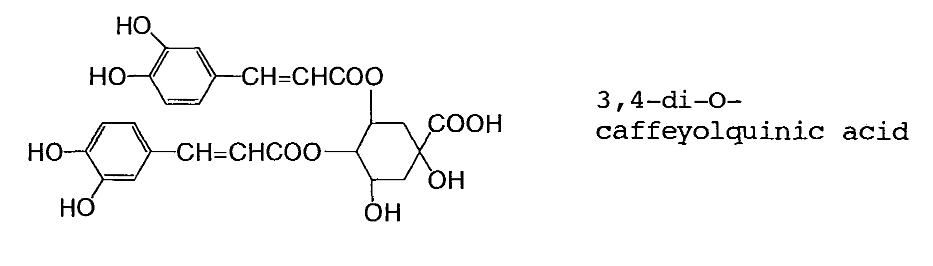

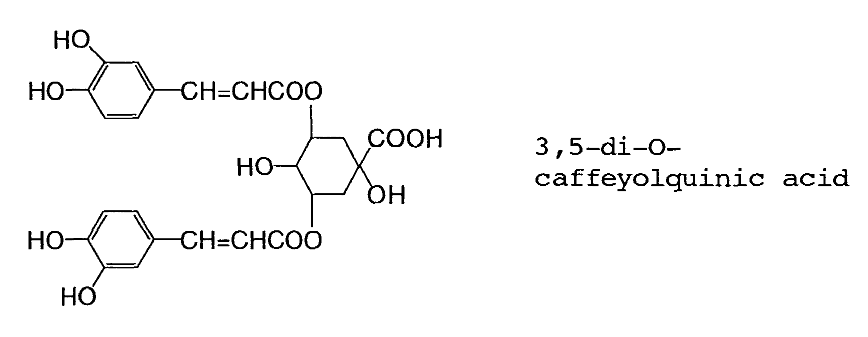

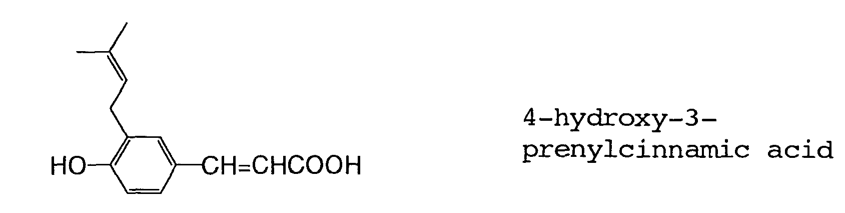

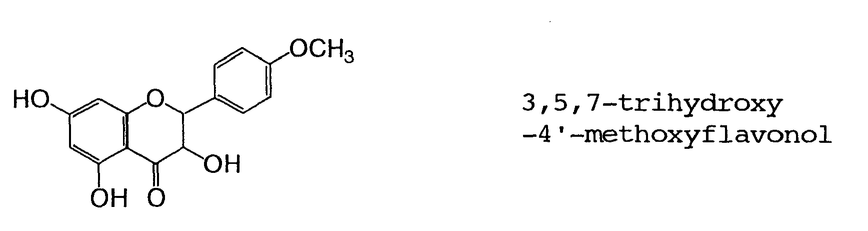

The active oxygen eliminator of the present invention may further comprise, as active components, 3,5-diprenyl-4-hydroxycinnamic acid (artepillin C), kaempheride, beturetol, chlorogenic acid, ermanin, chrysin, caffeic acid, kaempherol and vanillin, which have the following structures.

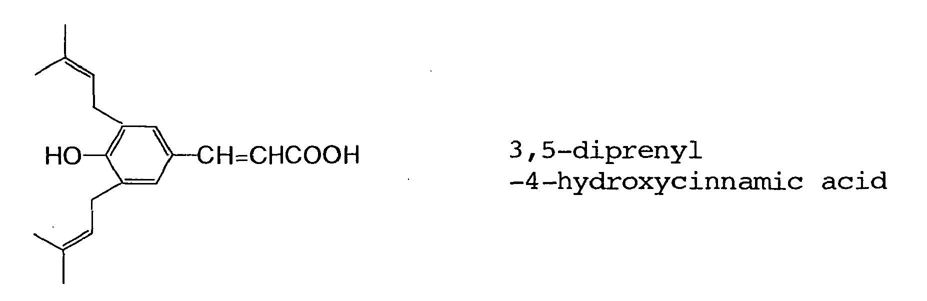

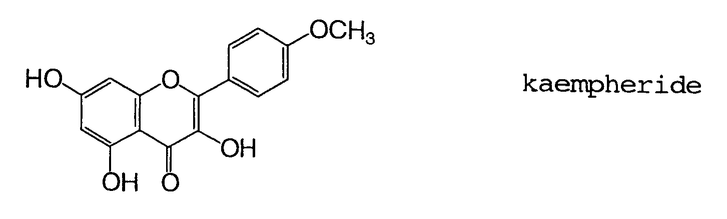

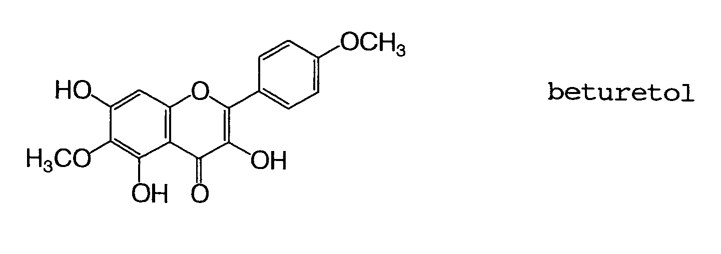

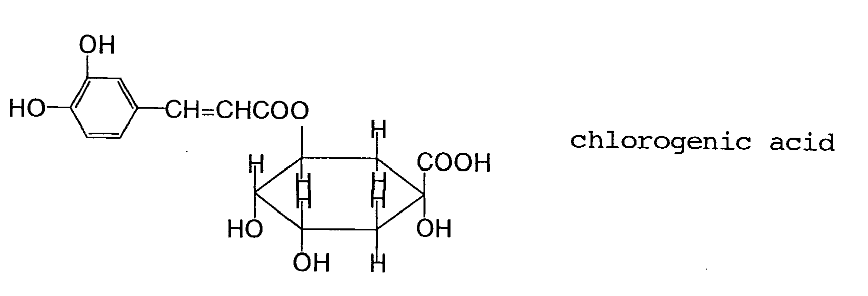

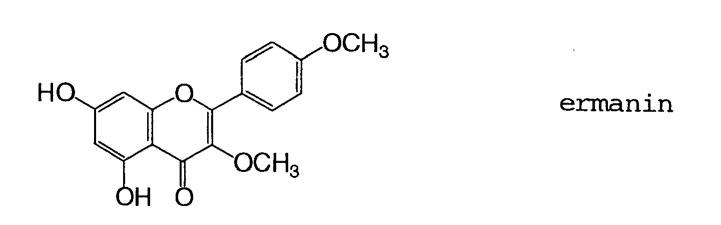

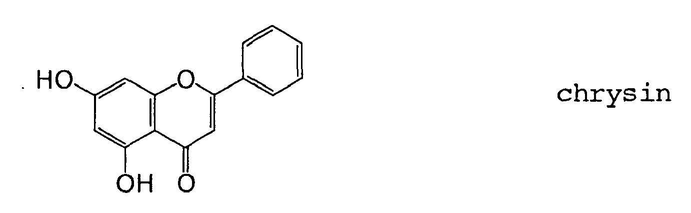

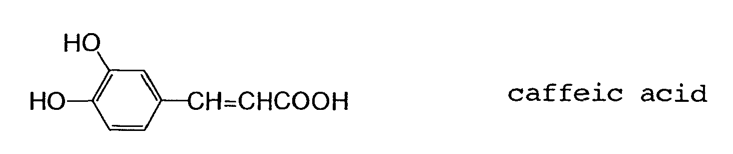

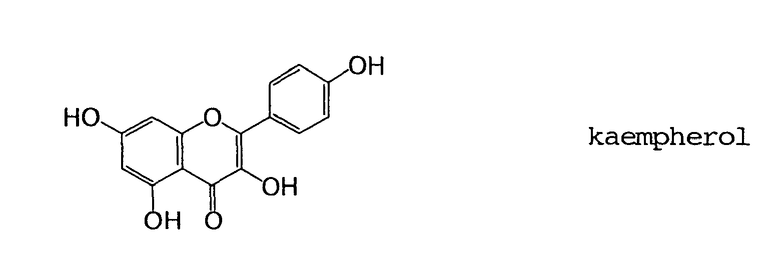

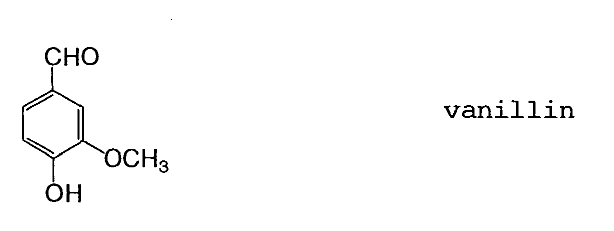

The above kaempheride refers to 4'-methoxy-3,5,7-trihydroxyflavone, the above beturetol refers to 4',6'-dimethoxy-3,5,7-trihydroxyflavone, the above ermanin refers to 3,4'-dimethoxy-5,7-dihydroxyflavone, the above chrysin refers to 5,7-dihydroxyflavone, and the above kaempherol refers to 3,4',5,7-tetrahydroxyflavone.

The organic solvent contained in the above active oxygen eliminator of the present invention is preferably a polyhydric alcohol compound having two or more hydroxyl groups in a molecule. Specific examples of the organic solvent include glycerin, ethylene glycol, diethylene glycol, propylene glycol, dipropylene glycol, an alkylene oxide adduct of glycerin, an alkylene oxide adduct of ethylene glycol, an alkylene oxide adduct of propylene glycol and gluconodeltalactone, and further include sugar alcohols such as xylitol, hexitol, mannitol and sorbitol. The active oxygen eliminator may contain one or more members of these organic solvents. When the active oxygen eliminator is used in foods, glycerin and various sugar alcohols are preferred.

Examples of the emulsifier include glycerin fatty acid ester, polyglycerin fatty acid ester, propylene glycol fatty acid ester, sorbitan fatty acid ester, a sucrose fatty acid ester, lecithins and saponins.

Examples of the above glycerin fatty acid ester include glycerin monolaurate, glycerin monopalmitate, glycerin monostearate, glycerin monooleate, glycerin monolinoleate and glycerin monoricinoleate.

Examples of the polyglycerin fatty acid ester include diglycerin monolaurate, diglycerin monopalmitate, diglycerin monostearate, diglycerin monooleate, diglycerin monolinoleate, diglycerin monoricinoleate, tetraglycerin monolaurate, tetraglycerin dilaurate, tetraglycerin monopalmitate, tetraglycerin dipalmitate, tetraglycerin monostearate, tetraglycerin distearate, tetraglycerin monooleate, tetraglycerin dioleate, tetraglycerin monolinoleate, tetraglycerin dilinoleate, tetraglycerin monoricinoleate, tetraglycerin diricinoleate, tetraglycerin monobehenate, tetraglycerin dibehenate, pentaglycerin monolaurate, pentaglycerin dilaurate, pentaglycerin monopalmitate, pentaglycerin dipalmitate, pentaglycerin monostearate, pentaglycerin distearate, pentaglycerin monooleate, pentaglycerin, dioleate, pentaglycerin monolinoleate, pentaglycerin dilinoleate, pentaglycerin monoricinoleate, pentaglycerin diricinoleate, pentaglycerin monobehenate, pentaglycerin dibehenate, decaneglycerin monolaurate, decaneglycerin dilaurate, decaneglycerin trilaurate, decaneglycerin monopalmitate, decaneglycerin dipalmitate, decaneglycerin tripalmitate, decaneglycerin monostearate, decaneglycerin distearate, decaneglycerin tristearate, decaneglycerin monooleate, decaneglycerin dioleate, decaneglycerin trioleate, decaneglycerin monolinoleate, decaneglycerin dilinoleate, decaneglycerin trilinoleate, decaneglycerin monoricinoleate, decaneglycerin diricinoleate, decaneglycerin triricinoleate, decaneglycerin monobehenate, decaneglycerin dibehenate, decaneglycerin tribehenate, decaneglycerin monoisostearate, decaneglycerin sesquiisostearate, decaneglycerin diisostearate, decaneglycerin triisostearate, decaneglycerin mono(12-hydroxy) stearate, decaneglycerin di(12-hydroxy) stearate and decaneglycerin tri(12-hydroxy) stearate.

Examples of the propylene glycol fatty acid ester include propylene glycol monolaurate, propylene glycol monopalmitate, propylene glycol monostearate, propylene glycol monooleate, propylene glycol monolinoleate, propylene glycol monoricinoleate, propylene glycol monoisostearate and propylene glycol mono(12-hydroxy) stearate.

Examples of the sorbitan fatty acid ester include sorbitan monolaurate, sorbitan dilaurate, sorbitan monopalmitate, sorbitan dipalmitate, sorbitan monostearate, sorbitan distearate, sorbitan monooleate, sorbitan dioleate, sorbitan monolinoleate, sorbitan dilinoleate, sorbitan monoricinoleate, sorbitan diricinoleate, sorbitan monobehenate, sorbitan dibehenate, sorbitan monoisostearate, sorbitan diisostearate, sorbitan mono(12-hydroxy) stearate and sorbitan di(12-hydroxy) stearate.

Examples of the sucrose fatty acid ester include sucrose monolaurate, sucrose dilaurate, sucrose monopalmitate, sucrose dipalmitate, sucrose monostearate, sucrose distearate, sucrose monooleate, sucrose dioleate, sucrose monolinoleate, sucrose dilinoleate, sucrose monoricinoleate, sucrose diricinoleate, sucrose monoisostearate, sucrose diisostearate, sucrose mono(12-hydroxy) stearate and sucrose di(12-hydroxy)stearate.

Examples of the lecithins include soybean lecithin and other plant lecithin, and examples of saponins include quillaiasaponin and soybean saponin.

The active oxygen eliminator may contain one or more members of these emulsifiers. Since the above emulsifiers are usable for foods, they may be contained in the active oxygen eliminator without any problem when the active oxygen eliminator is used for foods.

In the active oxygen eliminator of the present invention, the contents of water, the above organic solvent and the above emulsifier are as follows. When the total content of these is 100 mass%, generally, the content of water is 5 to 50 mass%, the content of the organic solvent is 45 to 94.9 mass%, and the content of the emulsifier is 0.1 to 10 mass%. Preferably, the content of water is 5 to 30 mass%, the content of the organic solvent is 65 to 94.5 mass%, and the content of the emulsifier is 0.1 to 5 mass%.

In the active oxygen eliminator of the present invention, the total content of the above active components is determined as required depending upon use, while it is generally approximately 3 to 40 mass%.

The active oxygen eliminator of the present invention can be produced, for example, by subjecting a raw propolis mass to extracting treatment.

Desirably, the raw propolis mass is used after it is kept at 15°C or lower, preferably, at a temperature in the range of approximately 0 to 10°C, so that it can be efficiently pulverized and subjected to the extracting treatment.

First, the above raw propolis mass is pulverized with a pulverizer. The diameter of openings of a screen fitted to the above pulverizer is generally 1 to 5 mm, preferably 2 - 3 mm. Then, an extracting solvent that is a mixture solution containing water, the above organic solvent and the above emulsifier having the above-described contents is added in an amount of approximately 3 to 7 parts by mass per part by mass of the pulverization product of the raw propolis mass. When the amount of the extracting solvent is less than 3 parts by mass, the efficiency of extraction is degraded due to the viscousness of the pulverization product of the raw propolis mass and the extracting solvent. When it exceeds 7 parts by mass, the content of the active components in an active oxygen eliminator produced may decrease.

Then, the mixture of the product of the raw propolis mass with the extraction solvent is heated to a temperature of approximately 50 to 90°C, preferably 65 to 80°C, and maintained at this temperature for approximately 30 to 120 minutes to carry out the extracting treatment. After the extracting treatment, a solid content is filtered off, to give the active oxygen eliminator of the present invention.

The thus-obtained active oxygen eliminator of the present invention may be concentration-adjusted by distilling extracting solvent off or by newly adding extracting solvent so that it has a concentration suitable for use. Further, the active oxygen eliminator may be used in the form of a powder obtained by freeze-drying or spray-drying.

The active oxygen eliminator of the present invention contains a broad range of components including hydrophilic and hydrophobic components, such as flavonoids, hydroxycinnamic acid derivatives, other phenolic compounds, and the like, has high capability of removing active oxygen and free radicals and has high antioxidation capability, and it can be used, for example, in foods and cosmetics and can be also used further in animal feeding stuffs.

The active oxygen eliminator of the present invention can be incorporated into compositions such as foods and cosmetics while it is in a state of a liquid product as it is. Alternatively, the active components of the above active oxygen eliminator may be formed as a preparation and the preparation may be incorporated into the above compositions. The compositions are therefore imparted with the active oxygen eliminating effect and antioxidation effect, so that the above compositions can be improved in commercial product value.

The preparation is obtained as follows. When the preparation is a tablet, capsule, powder or granule, it can be prepared and formulated by combining the above active components with starch, a vehicle such as lactose, mannitol, or the like, a binder such as carboxylmethyl cellulose, hydroxypropyl cellulose, or the like, a disintegrant such as crystalline cellulose, carboxylmethyl cellulose calcium, or the like and a lubricant such as talc, magnesium stearate, or the like, and optionally with a wetting agent, a colorant, perform, and the like. When the preparation is solution, it can be prepared in the form of an aqueous or oil-based emulsion or syrup. It can be prepared by incorporating a suspension-forming agent such as simple syrup, sorbitol, methyl cellulose, carboxylmethyl cellulose or the like or an emulsifier such as egg yolk lecithin, sorbitan monofatty acid ester, lauromacrogol, castor oil, or the like and optionally incorporating an antiseptic agent, a preserving agent, a stabilizer, or the like as required. When the preparation is ointment, it can be prepared by incorporating a hydrophobic base such as petrolatum, a paraffin, silicon, a plastic base, a plant oil, a wax, or the like, a hydrophilic base such as purified lanolin, a carboxyvinyl polymer, propylene glycol, 1,3-butanediol, or the like or an emulsifier such as polyethanolamine, or the like as required.

According to the present invention, there are also provided a food and cosmetic comprising the above active oxygen eliminator.

The type of the above food includes a type that the above active oxygen eliminator itself has, seasonings such as miso, soy sauce, mayonnaise, etc., cooking oils such as salad oil, etc., various cooked foods, sweets, ices, a candy, chewing gum, confectionery and a beverage such as fruit juice. These foods may contain any component depending upon consumptions. For example, ices and beverages may contain juice, a sweetener, an acidulant, a colorant and a flavor as required. The amount of the active components in the present invention may be changed as required depending upon types of foods.

Type of the cosmetic includes various types, such as facial washes including a lotion, a skin milk and a cream, a facial mask, a make-up cosmetic, a hair cosmetic, lip stick and cream, a nail cosmetic, a bath preparation, an antiperspirant, and the like. These cosmetics may contain any component as required. For example, a cream may contain a hydrophobic base such as petrolatum, a paraffin, squalane, or the like, a hydrophilic base such as lanolin, propylene glycol, 1,3-butanediol, or the like, an emulsifier such as a fatty acid monoglyceride, a sorbitan fatty acid ester, or the like, an antiseptic, a pigment, a perfume, or the like, and may optionally contain a nutritional supplement, a humectant, a whitening agent, an ultraviolet absorbent, or the like as required. These are incorporated according to conventional methods. Similarly, any other product may contain any necessary component as required depending upon its type. The amount of the active components in the present invention, which are added to cosmetics, may be changed as required depending upon types of the cosmetics.

The active oxygen eliminator of the present invention may be incorporated into animal feeding stuffs. The feeding stuffs include various types, and examples thereof include a powdered, paste and pelletized assorted feeds for livestock, poultry and fishes. These feeding stuffs may contain any component as required depending upon use. For example, the paste feeding stuff may contain a colorant, a flavor, etc., as required. The amount of the active components in the present invention, which are incorporated into the feeding stuffs, may be changed as required depending upon types thereof.

### Examples

The present invention will be explained further in detail with reference to Examples hereinafter, while the present invention shall not be limited by these Examples.

### Example 1

### (1) Extracts from raw propolis mass

As an extracting solvent, (a) water alone, (b) glycerin alone, (c) a mixture of 10 mass% of water with 90 mass% of glycerin and (d) a mixture of 10 mass% of water, 89 mass% of glycerin and 1 mass% of an emulsifier (glycerin monooleate) were used.

To 25 g of a pulverization product of a raw propolis mass (Baccharis dracunculifolia base type originated in Brazil) was added 200 g of one of the above extracting solvent (a) to (d), and the mixture was extracted at 70°C for 1 hour and filtered. In this matter, four raw propolis mass extracts (a), (b), (c) and (d) were obtained.

### (2) Propolis food standard component contents

Each of the four raw propolis mass extracts (a), (b), (c) and (d) obtained in the above (1) was measured for the Quercetin (supplied by Tokyo Kasei Co.Ltd) equivalent weight by an absorptiometric method (λ = 415 nm) according to the Propolis Food Standard Component Content Measurement Method (1995 Version) of a juridical foundation, Japan Health Food & Nutrition Food Association. Table 1 shows the results.

Further, they were measured for propolis food standard component contents (mass %) according to the Propolis Food Standard Content Measurement Method (1995 Version) of the juridical foundation, Japan Health Food & Nutrition Food Association. Table 1 shows the results.

### (3) DPPH radical eliminating activity

For evaluating the antioxidation activity of each of the four raw propolis mass extracts (a), (b), (c) and (d) obtained in the above (1), their ethanol solutions having various concentrations were prepared, and each of them was independently mixed with an equivalent amount of an ethanol solution containing DPPH (1,1-diphenyl-2-picrylhydrazyl) (supplied by Wako Pure Chemical Industries, Ltd.) having a concentration of 60 micromoles/liter. Each mixture was measured for an absorbance (λ = 520 nm) after 30 minutes, to determine IC₅₀ values that represented the elimination of DPPH radicals by the raw propolis mass extract. Table 1 shows the results.

**Table 1**

| Type of extracting solvent | Quercetin equivalent weight (mg) | Propolis standard component content (mass%) | Activity to eliminate DPPH radicals (%IC₅₀) |
|---|---|---|---|
| (a) | 180 | 5.3 | 0.057 |
| (b) | 321 | 9.7 | 0.021 |
| (c) | 298 | 9.4 | 0.020 |
| (d) | 353 | 11.0 | 0.020 |

It is seen from Table 1 that the raw propolis mass extract (d) (an active oxygen eliminator of the present invention) obtained by using the extracting solvent (d) containing a mixture of water, the organic solvent and the emulsifier has a higher propolis food standard component contents (Quercetin equivalent weight, mass %) than the raw propolis mass extracts (a), (b) and (c) obtained by using the other extracting solvents.

It is further seen that the raw propolis mass extract (d) exhibits DPPH radical elimination activity equivalent to those of the raw propolis mass extracts (b) and (c).

### (4) Analysis of active components by HPLC

The raw propolis mass extracts (a), (b), (c) and (d) obtained in the above (1) were analyzed by high-performance liquid chromatography (HPLC).

The measurement conditions by HPLC were as follows.

| | |
|---|---|
| Column | : CLC-ODS (150 x 6.0 mm, i.d.) |
| Column temp. | : 40°C |
| Mobile phase : | developing solutions |

| Linear gradient conditions: | |
|---|---|
| At initial time | |
| 0.5 % acetic acid (Wako Pure Chemical Industries, Ltd.)-methanol (Wako Pure Chemical Industries, Ltd.) (70:30 v/v) | |

| After 30 minutes | |
|---|---|
| 0.5 % acetic acid-methanol (20:80 v/v) | |
| Analysis time : | 50 minutes |
| Flow rate : | 1.0 ml/minute |
| Measurement wavelength λ : | 275 nm. |

The raw propolis mass extracts (a), (b), (c) and (d) were respectively dissolved in distilled water for HPLC (Wako Pure Chemical Industries, Ltd.) or methanol for HPLC (concentration of the extract: 10 mg/ml), filtered through Sartoriusminisarto (0.45 µm, Goettinggen, Germany), and 10 µl each of the filtrates were used for the HPLC analysis.

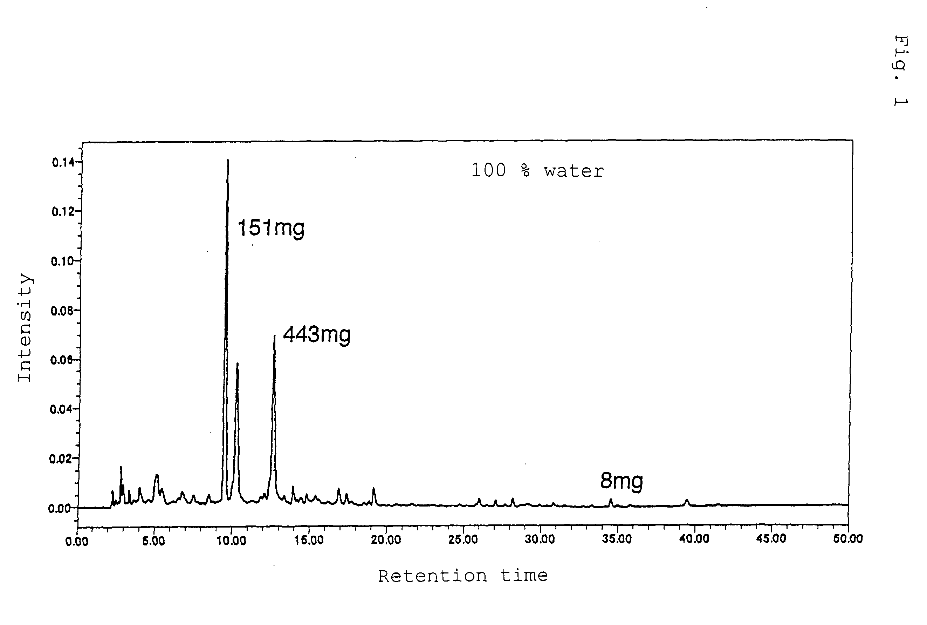

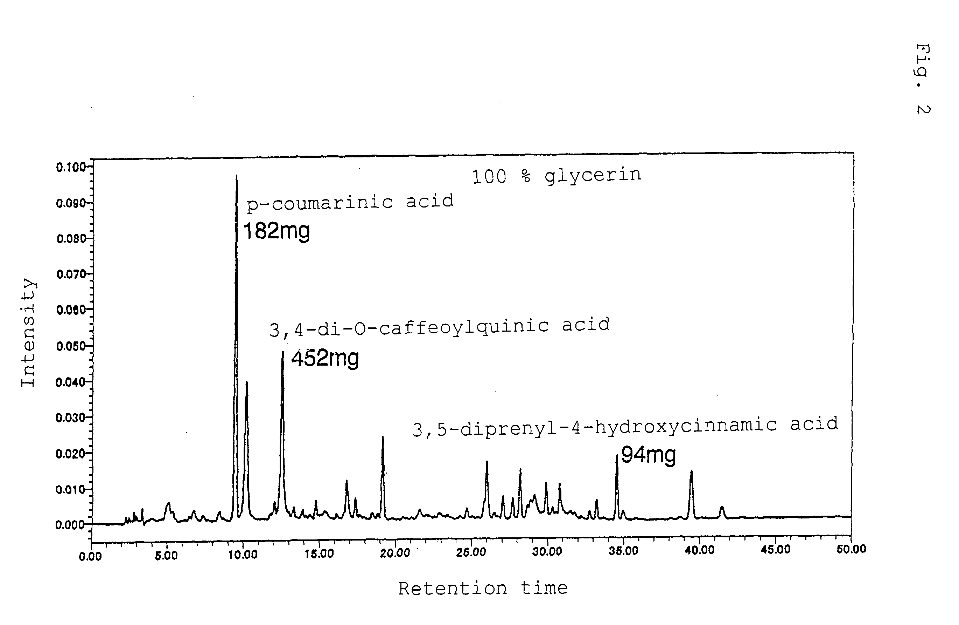

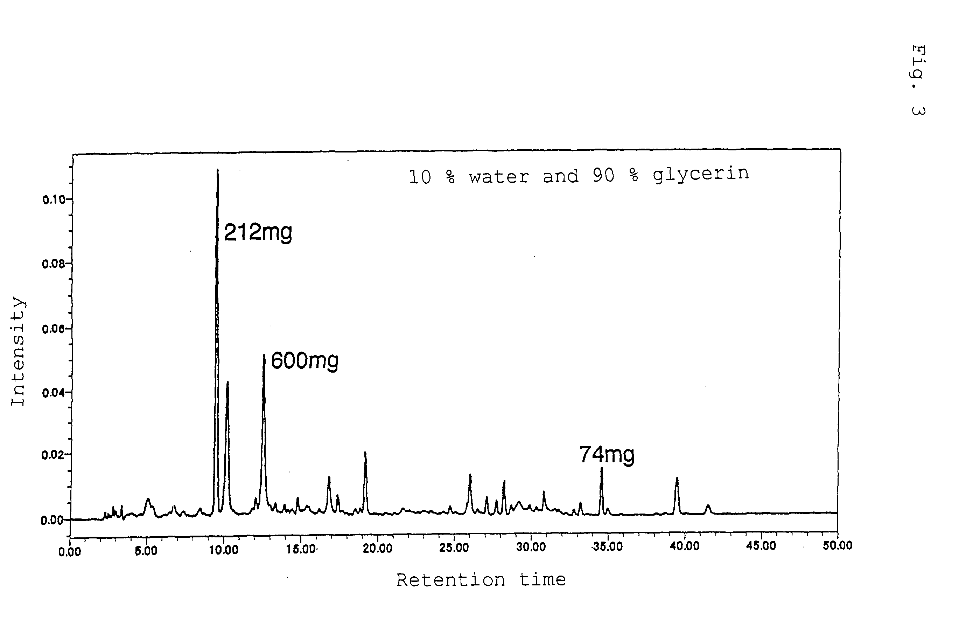

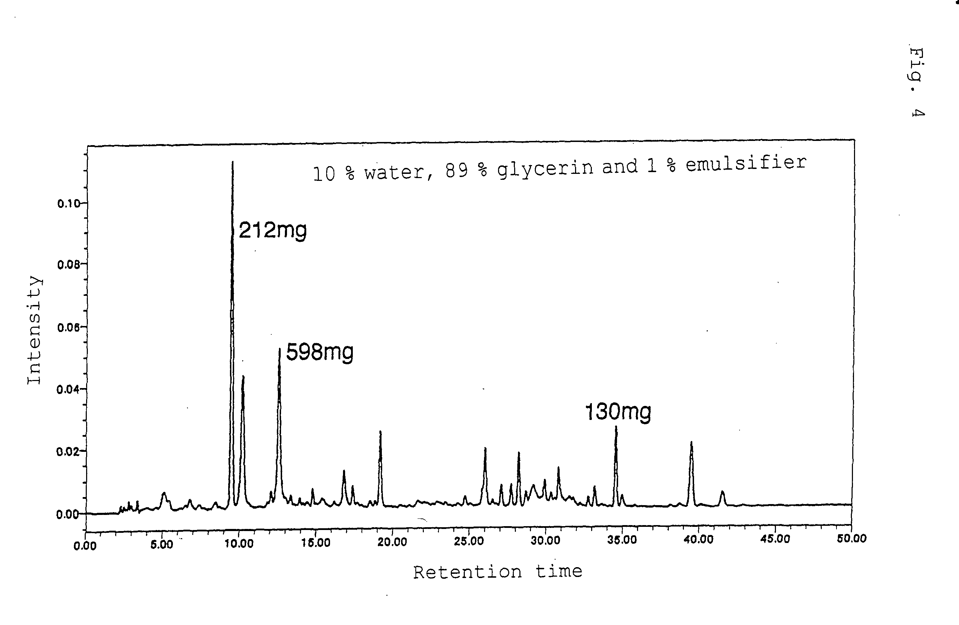

Figs. 1, 2, 3 and 4 show chromatograms of the raw propolis mass extracts (a), (b), (c) and (d), respectively, and Table 2 shows corresponding peak areas and peak heights while using retention times of the chromatograms as an indexes.

**Table 2**

| CO | R.T. | Extract (a) | | Extract (b) | | Extract (c) | | Extract (d) | |
|---|---|---|---|---|---|---|---|---|---|
| | | Peak area | Peak height | Peak area | Peak height | Peak area | Peak height | Peak area | Peak height |
| a | 5.1 | 125290 | 6068 | 102569 | 4791 | 118675 | 5196 | 116167 | 5088 |
| b | 5.4 | 64218 | 3516 | 46914 | 2708 | 48786 | 3089 | 49486 | 3048 |
| c | 6.8 | 67983 | 2923 | 49105 | 2668 | 62464 | 3141 | 62474 | 3148 |
| d | 8.5 | 36815 | 2422 | 28592 | 2137 | 46542 | 2485 | 45092 | 2452 |
| e | 9.5 | 613175 | 63510 | 736791 | 79462 | 857236 | 90101 | 858366 | 90377 |
| f | 10.2 | 452055 | 26322 | 485907 | 31914 | 599082 | 35311 | 591450 | 35039 |
| g | 12.1 | 30527 | 2569 | 42026 | 4122 | 55639 | 4936 | 58157 | 5020 |
| h | 12.5 | 470342 | 31374 | 551542 | 39007 | 637013 | 42228 | 634979 | 41826 |
| i | 13.5 | | | 37333 | 2824 | 47636 | 3484 | 51477 | 3869 |
| j | 14.0 | | | 24799 | 2254 | 44366 | 3166 | 33045 | 3091 |
| k | 14.8 | | | 48871 | 4511 | 66827 | 4881 | 54559 | 5328 |
| 1 | 15.4 | | | 22556 | 1933 | 92674 | 2938 | 92312 | 2965 |
| m | 16.8 | 60700 | 3357 | 131557 | 9200 | 163546 | 10024 | 165998 | 10080 |
| n | 17.4 | | | 5020-1 | 4998 | 63077 | 5497 | 69077 | 6073 |
| o | 19.2 | 43709 | 3403 | 185670 | 19187 | 172834 | 16222 | 212072 | 20240 |
| p | 21.6 | | | 49904 | 2512 | 49549 | 1957 | 51501 | 2031 |
| q | 26.0 | | | 183990 | 13576 | 149566 | 10422 | 215098 | 15207 |
| r | 27.1 | | | 55024 | 5542 | 51095 | 4697 | 62991 | 5840 |
| s | 27.7 | | | 54329 | 5180 | 45326 | 3843 | 65628 | 5931 |
| t | 28.2 | | | 115784 | 11797 | 89560 | 8834 | 139402 | 14079 |
| u | 29.1 | | | 123128 | 5785 | 104342 | 3467 | 166476 | 5674 |
| v | 29.9 | | | 94387 | 8707 | 54525 | 2650 | 115563 | 7012 |
| w | 30.8 | | | 115012 | 8519 | 74578 | 6122 | 154801 | 10109 |
| x | 31.5 | | | 43888 | 2120 | 27047 | 1620 | 47771 | 2711 |
| y | 33.2 | | | 42724 | 4575 | 31290 | 3187 | 51312 | 5149 |
| z | 34.5 | | | 132448 | 14993 | 104284 | 11750 | 183655 | 20617 |
| AA | 39.4 | | | 154791 | 11187 | 126105 | 9084 | 232224 | 16489 |
| AB | 41.5 | | | 44139 | 2726 | 34879 | 2154 | 61039 | 3745 |

| | | | | | | | | | |
|---|---|---|---|---|---|---|---|---|---|
| Notes: No data of peak area of 30,000 or less is shown. | | | | | | | | | |
| CO = Compound, R.T. = Retention time (minute) | | | | | | | | | |

Compound names are as follows: a: chlorogenic acid, c: caffeic acid, d: vanillin, e: p-coumarinic acid, f: 3,5-di-O-caffeoylquinic acid, h: 3,4-di-O-caffeoylquinic acid, o: 3,5,7-trihydroxy-4'-methoxyflavonol. p: kaempherol, q: 4-hydroxy-3-prenylcinnamic acid, s: chrysin, u: kaempheride, z: 3,5-diprenyl-4-hydroxycinnamic acid (artepillin C),

As shown in Figs. 1 to 4, it is seen that peaks that are not observed in the chromatogram of the raw propolis mass extract (a) appear in the chromatograms of the raw propolis mass extracts (b), (c) and (d) with an increase in the ratio of methanol of the mobile phase as compared with the raw propolis mass extract (a), and further comparisons based on individual peaks shows that peak groups of compounds that are considered to exhibit hydrophobicity appear on the chromatograms of the raw propolis mass extracts (b), (c) and (d), which endorses the results shown in the above (2) and (3). More specifically, it is confirmed that the peak areas and peak heights of the hydrophilic compounds a to h in the four raw propolis mass extracts are nearly at the same level or that they tend to be predominant in the raw propolis mass extract (d). Further, with regard to the compounds i to AB, they are clearly confirmed on the chromatograms of the raw propolis mass extracts (b), (c) and (d) except the compounds m, o (3,5,7-trihydroxy-4'-methoxyflavonol) and z (3,5-diprenyl-4-hydroxycinnamic acid), while it is confirmed that they tend to be predominant in the raw propolis mass extract (d).

More specifically, when the raw propolis mass extracts (b), (c) and (d) are compared with regard to physiologically active components u (kaempheride) and z (3,5-diprenyl-4-hydroxycinnamic acid) which appear after a retention time of 25 minutes while keeping constant the measurement values of physiologically active substances a (chlorogenic acid), c (caffeic acid), f (3,5-di-O-caffeoylquinnic acid), h (3,4-di-O-caffeoylquinnic acid), etc., which appear before a retention time of 15 minutes, it is confirmed that the measurement values clearly increase in the raw propolis mass extract (d). It is therefore considered that an increase in the content of the compounds u and z contributes to an increase in the measurement values in the above (2), and it is suggested that the raw propolis mass extract (d) (the active oxygen eliminator of the present invention) contains a wide range of components that range from hydrophilic components to hydrophobic components, and has promising physiological activities including the capability of eliminating radicals.

### Industrial Utility

The active oxygen eliminator of the present invention has high safety to human bodies, has the capability of highly eliminating active oxygen and free radicals and high antioxidation capability, and can be suitably used in foods, cosmetics, animal feeding stuffs, and the like.

## Claims

1. An active oxygen eliminator derived from a natural substance, which comprises water, an organic solvent and an emulsifier and also comprises, as essential active components, p-coumarinic acid, 3,4-di-O-caffeoylquinic acid, 3,5-di-O-caffeoylquinic acid, 4-hydroxy-3-prenylcinnamic acid and 3,5,7-trihydroxy-4'-methoxyflavonol.

2. The active oxygen eliminator derived from a natural substance as claimed in claim 1, which further comprises, as active components, 3,5-diprenyl-4-hydroxycinnamic acid, kaempheride, beturetol, chlorogenic acid, ermanin, chrysin, caffeic acid, kaempherol and vanillin.

3. The active oxygen eliminator derived from a natural substance as claimed in claim 1 or 2, wherein the organic solvent is a polyhydric alcohol compound having 2 or more hydroxyl groups in a molecule.

4. The active oxygen eliminator derived from a natural substance as claimed in claims 1, 2 or 3, wherein the emulsifier is at least one member selected from a glycerin fatty acid ester, a polyglycerin fatty acid ester, a propylene glycol fatty acid ester, a sorbitan fatty acid ester, a sucrose fatty acid ester, lecithins and saponins.

5. A food comprising the active oxygen eliminator derived from a natural substance recited in any one of claims 1 to 4.

6. A cosmetic comprising the active oxygen eliminator derived from a natural substance recited in any one of claims 1 to 4.
